# EUROPEAN PATENT SPECIFICATION

(11) **EP 2 404 585 B1**
(45) Date of publication and mention of the grant of the patent: **31.12.2014**
(21) Application number: 10748642.5
(22) Date of filing: 23.02.2010
(51) Int. Cl.: A61F 13/32, A61F 13/20, A61F 13/30

(54) **TAMPON**
STOPFEN
TAMPON HYGIÉNIQUE

(30) Priority: 02.03.2009 JP 2009048394
(43) Date of publication of application: 11.01.2012
(73) Proprietor: Uni-Charm Corporation, Ehime 799-0111 (JP)
(72) Inventor: WADA, Mitsuhiro, Kanonji-shi Kagawa 769-1602 (JP); NOZAKI, Satoshi, Kanonji-shi Kagawa 769-1602 (JP)
(74) Representative: Eke, Philippa Dianne
(86) International application number: PCT/JP2010/052737
(87) International publication number: WO 2010/101046

(56) References cited:
- WO-A1-89/04159
- JP-A- S6 294 156
- JP-A- H06 315 505
- JP-A- H10 513 084
- JP-A- 2005 530 557
- JP-U- 3 094 218
- JP-U- S6 215 331
- JP-U- S63 169 126
- US-A- 3 015 332
- US-A- 3 433 225
- US-A- 3 717 149
- US-A- 4 536 178
- US-A1- 2003 236 485

## Description

### Technical Field

The present invention relates to a tampon.

### Background Art

Tampons having a tampon main body; an accommodating cylinder that accommodates the tampon main body, the accommodating cylinder having an opening at a leading end; and a pushing member that pushes the tampon main body outside of the accommodating cylinder from the opening by moving inside the accommodating cylinder, are widely known.

Then, by a user of the tampon handling the pushing member, the tampon main body is pushed outside from the opening, and the tampon main body is inserted into a vagina.

PCT Application No. WO 89/04159 (Patent Literature 2) relates to a tampon inserting device, US Patent No. 3,717,149 (Patent Literature 3) discloses a injector package for a catamenial tampon, and US patent Application No. US 2003/0236485 (Patent Literature 4) describes a tampon applicator having corrugated grip.

### Citation List

### Patent Literature

Patent Literature 1: Japanese Laid-Open Patent Application No. Hei5-84263
Patent Literature 2: PCT Application No. WO 89/04159
Patent Literature 3: US Patent No. US 3,717,149
Patent Literature 4: US Patent Application No. US 2003/236485

### Summary of Invention

### Technical Problem

In the prior art, at the time the tampon main body was to be pushed outside of the accommodating cylinder by the user handling the pushing member, there was a case where the tampon main body was not pushed outside in a satisfactory manner. Therefore, there is a demand for a tampon in which the tampon main body is pushed outside in a satisfactory manner.

The present invention has been made in view of such a disadvantage and the object of the present invention is to obtain a tampon in which the tampon main body is pushed outside in a satisfactory manner.

### Solution to Problem

In order to achieve the above-mentioned object, the present invention provides the tampon of independent Claim 1. The dependent claims specify preferred but optional features. The present invention comprises:
a tampon including:
   a tampon main body;
   an accommodating cylinder that accommodates the tampon main body, the accommodating cylinder having an opening at a leading end; and
   a pushing member that pushes out the tampon main body outside of the accommodating cylinder from the opening, by moving inside the accommodating cylinder,
   wherein the accommodating cylinder has ribs along a longitudinal direction of the accommodating cylinder on an inner surface of the accommodating cylinder.

Other features of the present invention will become apparent from descriptions of this specification and of accompanying drawings.

### Advantageous Effects of Invention

According to this invention, a tampon in which the tampon main body is pushed outside in a satisfactory manner is obtained.

### BRIEF DESCRIPTION OF DRAWINGS

Fig. 1 is a cross-sectional view showing elements of a tampon 10.
Fig. 2 is a cross-sectional view showing elements of the tampon 10.
Fig. 3 is an external view of an outer cylinder 40.
Fig. 4 is a diagram showing the outer cylinder 40 shown in Fig. 3 from the leading end side.
Fig. 5 is an A-A cross sectional view of Fig. 1 or Fig. 2.
Fig. 6 is a partial enlarged view of Fig. 2.
Fig. 7 is a diagram showing a tampon 10 according to a comparative example.
Fig. 8 is a diagram showing longitudinal ribs 54 according to another embodiment.
Fig. 9 is a diagram showing longitudinal ribs 54 according to yet another embodiment.
Fig. 10 is a diagram partially showing a tampon 10that does not form an embodiment of the claimed invention.

### Description of Embodiments

At least the following matters are disclosed in the present specification and accompanying drawings.

First, a tampon is provided including:
a tampon main body;
an accommodating cylinder that accommodates the tampon main body, the accommodating cylinder having an opening at a leading end; and
a pushing member that pushes out the tampon main body outside of the accommodating cylinder from the opening, by moving inside the accommodating cylinder,
wherein the accommodating cylinder has ribs along a longitudinal direction of the accommodating cylinder on an inner surface of the accommodating cylinder.

With such a tampon, a tampon is obtained in which the tampon main body is pushed outside in a satisfactory manner.

Further provided is the above-mentioned tampon:
wherein the accommodating cylinder preferably has greater than or equal to three ribs arranged at equal spacing in an inner peripheral direction of the inner surface.

With such a tampon, the tampon main body is to be pushed outside in a more satisfactory manner.

Further provided is the above-mentioned tampon,
wherein the tampon main body is preferably in contact with only the ribs, of the inner surface and the ribs.

With such a tampon, the tampon main body is to be pushed outside in a more satisfactory manner.

Further provided is the above-mentioned tampon,
wherein the accommodating cylinder preferably has the ribs at least at the leading end side from the center in the longitudinal direction of the accommodating cylinder.

With such a tampon, the tampon main body is to be pushed outside in a more satisfactory manner.

Further provided is the above-mentioned tampon:
wherein the accommodating cylinder preferably has a plurality of petaloid portions surrounding the opening, the accommodating cylinder not having the ribs on the petaloid portions.

With such a tampon, a problem that the opening is hard to open does not occur, and the tampon main body is to be pushed outside in a more satisfactory manner.

Further provided is the above-mentioned tampon,
wherein the accommodating cylinder includes
a major diameter portion positioned at the leading end side, the major diameter portion being formed with the opening, and
a minor diameter portion positioned at a rear end side opposite to the leading end side, the minor diameter portion having an internal diameter smaller than that of the major diameter portion,
the tampon main body is accommodated in only the major diameter portion, of the major diameter portion and the minor diameter portion,
the pushing member pushes out the tampon main body outside of the accommodating cylinder from the opening, by moving inside the major diameter portion and the minor diameter portion of the accommodating cylinder, and
the accommodating cylinder preferably has the ribs in only the major diameter portion, of the major diameter portion and the minor diameter portion.

With such a tampon, a satisfactory pushing out of the tampon main body can be efficiently performed.

Further provided is the above-mentioned tampon:
wherein a projection height of the ribs in a radial direction of the major diameter portion is preferably smaller than an internal diameter difference between the major diameter portion and the minor diameter portion.

With such a tampon, a problem that the tampon main body accommodated in the major diameter portion enters the minor diameter portion occurs is to be prevented.

Further, a tampon is provided:
wherein the accommodating cylinder includes
   a major diameter portion positioned at the leading end side, the major diameter portion being formed with the opening, and
   a minor diameter portion positioned at a rear end side opposite to the leading end side, the minor diameter portion having an internal diameter smaller than that of the major diameter portion,
   the tampon main body is accommodated in only the major diameter portion, of the major diameter portion and the minor diameter portion,
   the pushing member pushes out the tampon main body outside of the accommodating cylinder from the opening, by moving inside the major diameter portion and the minor diameter portion of the accommodating cylinder, and
   the accommodating cylinder preferably has the ribs in both the major diameter portion and the minor diameter portion.

With such a tampon, it is possible to make the pushing member move in a satisfactory manner.

Further provided is the above-mentioned tampon:
wherein a product is applied on an outer surface of the tampon main body.

With such a tampon, there occurs an effect that a problem that an amount of the product to be administered into the vaginal cavity lessens is prevented.

### === Structure of tampon ===

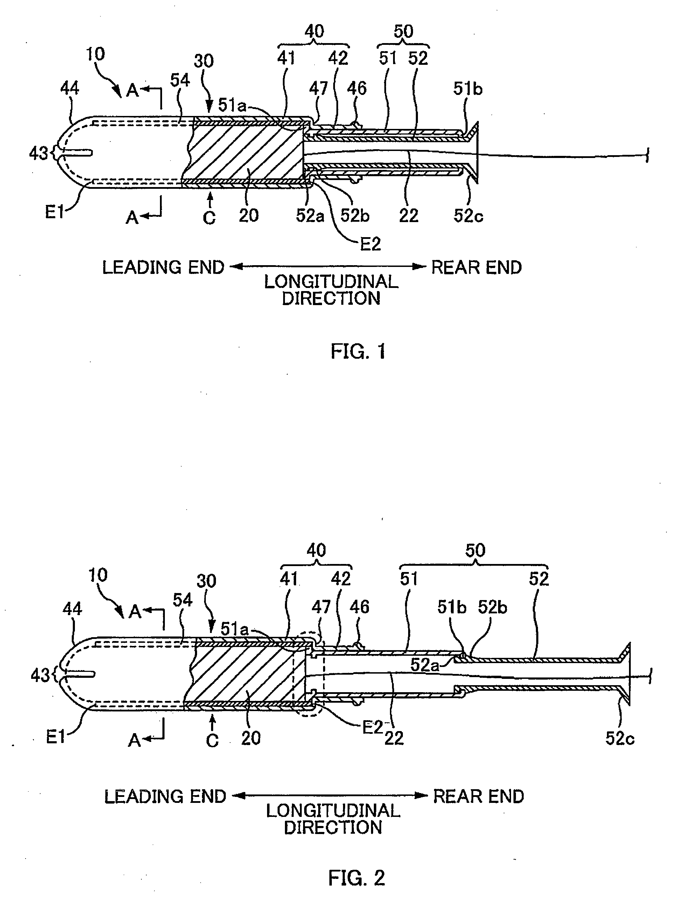

Structure of a tampon 10 will first be described with reference to Figs. 1 to 6.

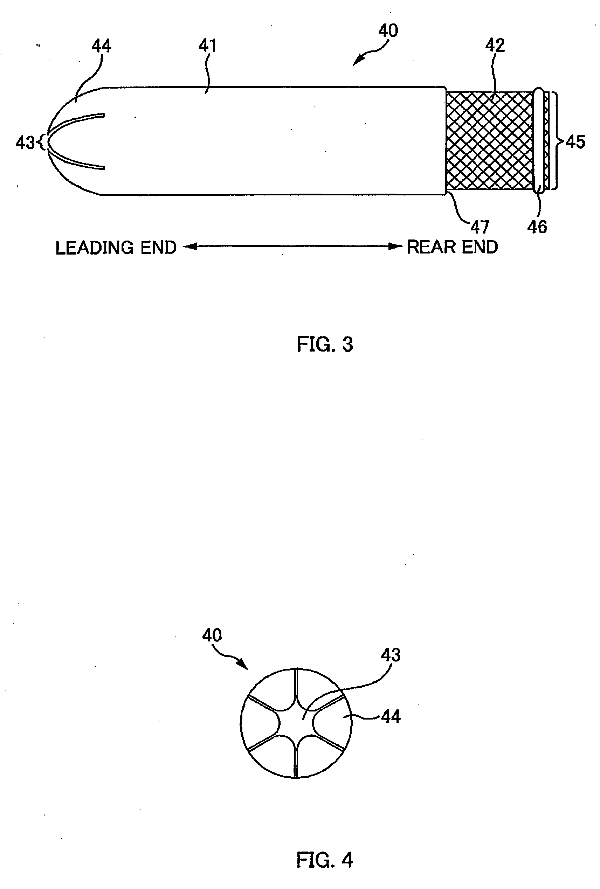

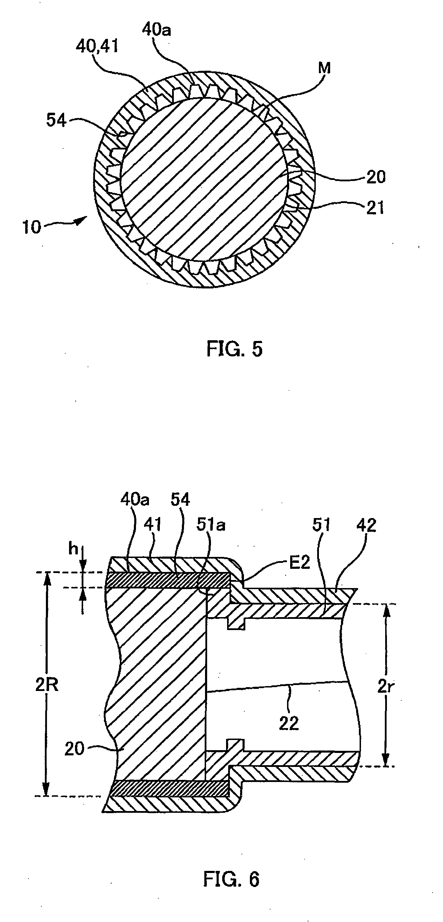

Figs. 1 and 2 are cross-sectional views illustrating components of a tampon 10. Fig. 1 shows the tampon 10 in a state where an inner cylinder 50 is contracted and Fig. 2 shows the tampon 10 in a state where the inner cylinder 50 is extended. Fig. 3 is an external view of an outer cylinder 40. Fig. 4 is a diagram showing the outer cylinder 40 of Fig. 3 from its leading end side. Fig. 5 is an A-A cross sectional view of Fig. 1 and Fig. 2. Fig. 6 is a partially enlarged view of Fig. 2. In the following description, along a longitudinal direction of the tampon 10, a side that is inserted into a vagina will be referred to as a leading end and an opposite side will be referred to as a rear end.

As shown in Fig. 1 and Fig. 2, the tampon 10 of the present embodiment has a tampon main body 20 and an applicator 30.

The tampon main body 20 is an absorbent body that blocks a vaginal cavity and absorbs liquid such as menstrual blood. The tampon main body 20 is an absorbent main body made of a rayon fiber (cotton body) covered with a covering body made of a polyester spun-bonded nonwoven fabric, and has a substantially bullet like shape.

Further, in this embodiment, a product M is applied on an outer surface 21 of the tampon main body 20. This product M is administered into the vaginal cavity, and is a mixture of a pine bark extract (Flavengenol of Toyo Shinyaku Co., Ltd.) and a polyethylene glycol. The pine bark extract exerts for example an antioxidant action, an anti-inflammatory action, an antimicrobial action, an antiviral action, an anti-allergic action, a deodorizing action, a vasodilatation action, or a lipoperoxidation inhibiting action. A mixing ratio of the pine bark extract and the polyethylene glycol is 1:9 (namely, the pine bark extract is 10 weight %, and the polyethylene glycol is 90 weight %). Note that, in this embodiment, the product M is coated on an entire outer surface of the tampon main body in a uniform manner with an approximately 0.1 mm thickness.

Further, the tampon main body 20 according to this embodiment is attached (sewn) with an extraction cord 22 as an example of a cord. This extraction cord 22 is a twine made of cotton. The extraction cord 22 extends from a rear end side of the tampon main body 20 and the extraction cord 22 is held by a user at the time of pulling out the tampon main body 20 from inside the vaginal cavity to outside the vaginal cavity. Further, as shown in Fig. 1 and Fig. 2, the extraction cord 22 extends through the applicator 30 and is somewhat extended out from a rear end of the applicator 30. Namely, a portion of the extraction cord 22 is exposed outside from the rear end of the applicator 30.

The applicator 30 is an aid device for facilitating the guiding of the tampon main body 20 into the vaginal cavity. As shown in Fig. 1 and Fig. 2, the applicator 30 includes the outer cylinder 40 that is an example of an accommodating cylinder and the inner cylinder 50 that is an example of the pushing member.

The outer cylinder 40 is for accommodating the tampon main body 20. This outer cylinder 40 is a cylindrical body made of a thermoplastic resin (in this embodiment, a polyethylene resin) by injection molding and has a satisfactory flexibility. The outer cylinder 40 includes a major diameter portion 41 positioned at the leading end side (that is, one end side in a longitudinal direction of the outer cylinder 40) and a minor diameter portion 42 positioned at the rear end side opposite to the leading end side (that is, another end side in the longitudinal direction of the outer cylinder 40), the minor diameter portion 42 having an internal diameter smaller than that of the major diameter portion 41 (Note that, the major diameter portion 41 has an external diameter that is greater than that of the minor diameter portion 42.). Thus, the external diameter (internal diameter) of the leading end portion of the outer cylinder 40 is greater than that of the rear end portion. As a result, an annular stepped portion 47 is formed between the major diameter portion 41 and the minor diameter portion 42.

The major diameter portion 41 is a portion of the outer cylinder 40 that serves mainly to accommodate the tampon main body 20 inside. That is, with the tampon 10 according to this embodiment, the tampon main body 20 is accommodated in, of the major diameter portion 41 and the minor diameter portion 42, only the major diameter portion 41 (Thus, a length of the major diameter portion 41 in the longitudinal direction of the outer cylinder 40 is made longer than a length of the tampon main body 20 in the longitudinal direction.). Further, the major diameter portion 41 is a portion that is inserted into the vaginal cavity with the tampon main body 20 being accommodated therein at the time of using the tampon 10.

The major diameter portion 41 (the outer cylinder 40) includes an opening at the leading end (Hereinbelow, referred to as "a leading end opening 43") and further includes a plurality of petaloid portions 44 (in the present embodiment, six petaloid portions 44) surrounding the leading end opening 43. Each of the plurality of petaloid portions 44 is inwardly bent in an arc in the radial direction of the outer cylinder 40 as shown in Fig. 3. Therefore, at the time the outer cylinder 40 is inserted into the vaginal cavity, the leading end portion of the outer cylinder 40 is substantially hemispherical as shown in Figs. 1 and 2, and the leading end opening 43 is in a substantially closed state as shown in Fig. 4. Further, at the time the tampon main body 20 is to be pushed out from the leading end opening 43 by an inner cylinder 50 to be described later, the leading end opening 43 is to be opened.

The minor diameter portion 42 is a portion of the outer cylinder 40 that serves mainly to provide a space in which a later-described inner cylinder 50 moves (However, of course, the inner cylinder 50 does not move in just the minor diameter portion 42, but also moves in the major diameter portion 41.). The minor diameter portion 42 is a grip portion held by a user when using the tampon 10.

Further, as shown in Fig. 3, the minor diameter portion 42 (the outer cylinder 40) includes an opening at a rear end (hereinbelow, referred to as a rear end opening 45) and also an annular rib 46 is provided, formed slightly nearer to the leading end side than the rear end opening 45.

Further, as shown in Figs. 1, 2, 5, and 6, the outer cylinder 40 is provided with ribs (hereinbelow, referred to as longitudinal ribs 54) on its (the outer cylinder 40's) inner surface 40a, the ribs being provided along the longitudinal direction of the outer cylinder 40. The outer cylinder 40 according to this embodiment is provided with the longitudinal ribs 54 at least at the leading end side from the center C (refer to Fig. 1) in the longitudinal direction of the outer cylinder 40. Further, in this embodiment, the outer cylinder 40 is provided with the longitudinal ribs 54 in only the major diameter portion 41, of the major diameter portion 41 and the minor diameter portion 42.

As shown in Fig. 5, thirty-two longitudinal ribs 54 are arranged at equal spacing in an inner peripheral direction of the inner surface 40a. That is, the outer cylinder 40 (major diameter portion 41) is provided with equal to or more than three longitudinal ribs 54 arranged at equal spacing in the inner peripheral direction of the inner surface 40a. Note that, in this embodiment, the longitudinal ribs 54 that are next to each other are not in contact with each other.

As shown in Figs. 1 and 2, each of the longitudinal ribs 54 is formed in a linear shape from the leading end side to the rear end side of the major diameter portion 41. More specifically, the longitudinal ribs 54 according to this embodiment are provided on the one hand up to the rearmost end E2 of the major diameter portion 41, but on the other hand are not provided up to the foremost end of the major diameter portion 41. That is, the longitudinal ribs 54 are not provided in the petaloid portions 44, and are provided up to the foremost end E1 of the major diameter portion 41 excluding the petaloid portions.

As shown in Fig. 5, furthermore, the longitudinal ribs 54 according to this embodiment are ribs projecting out in a radial direction of the major diameter portion 41, and the width of the rib becomes narrower as it gets closer to the center in the radial direction. Then, as shown in Fig. 6, a projection height h of the longitudinal ribs 54 in the radial direction of the major diameter portion 41 is made smaller than a difference between the internal diameters of the major diameter portion 41 and the minor diameter portion 42 (inner radius R of the major diameter portion 41 - inner radius r of the minor diameter portion = R-r) (h<R-r). In other words, the internal diameter of the major diameter portion 41 taking into consideration the longitudinal ribs 54 R-h (hereinbelow, referred to as a rib-considering internal diameter, for the sake of convenience) is made larger than an internal diameter r of the minor diameter portion (r<R-h).

Then, the tampon main body 20 has an external diameter substantially similar to the rib-considering internal diameter, and as shown in Fig. 5, the tampon main body 20 is accommodated in the major diameter portion 41 of the outer cylinder 40 in a state contacting, of the inner surface 40a and the longitudinal ribs 54, only the longitudinal ribs 54. That is, the outer surface 21 of the tampon main body 20 is in contact with only the leading end portions projecting in the radial direction of the longitudinal ribs 54, and is not in contact with the inner surface 40a.

The inner cylinder 50 serves to push out the tampon main body 20 outside of the outer cylinder 40 from the leading end opening 43, by moving inside the outer cylinder 40. This inner cylinder 50 is inserted into the outer cylinder 40, and is positioned at the rear end side of the tampon main body 20 in the outer cylinder 40. Then, the inner cylinder 50 travels along the outer cylinder 40 longitudinally, and pushes the tampon main body 20 from the rear towards the leading end opening 43. Thereby, the tampon main body 20 pushes each of the plurality of petaloid portions 44 outwardly in the radial direction of the outer cylinder 40 (i.e., opens the leading end opening 43), and is pushed out of the outer cylinder 40. In other words, the inner cylinder 50 is movable in the outer cylinder 40 and has a function of pushing the tampon main body 20 out of the outer cylinder 40.

Further, the inner cylinder 50 according to the present embodiment has a telescopic structure in order to provide a shorter overall length of the tampon 10 to make it compact. In detail, when the inner cylinder 50 is retracted as shown in Fig. 1, the length of the inner cylinder 50 becomes shorter than the length of the outer cylinder 40 and becomes a length that is convenient for carrying. On the other hand, when the inner cylinder 50 extends as shown in Fig. 2, the length of the inner cylinder 50 becomes a length that is sufficient to push the tampon main body 20 out of the outer cylinder 40. In the present embodiment, in order to achieve the telescopic structure of the inner cylinder 50, the inner cylinder 50 has a two-tier structure. In detail, as shown in Figs. 1 and 2, the inner cylinder 50 of the present embodiment includes the first inner cylinder 51 and the second inner cylinder 52 that is slidably inserted into the first inner cylinder 51.

The first inner cylinder 51 is a cylindrical body made of plastic by injection molding. The first inner cylinder 51 has an external diameter that is slightly smaller than an internal diameter of the minor diameter portion 42 of the outer cylinder 40. As shown in Fig. 1, the first inner cylinder 51 is slidably inserted into the minor diameter portion 42. An annular flange portion 51a is formed on an outer peripheral surface of the leading end part of the first inner cylinder 51. The flange portion 51a has an external diameter that is slightly smaller than the rib-considering internal diameter of the major diameter portion 41 of the outer cylinder 40 and engages an inner wall of the stepped portion 47, to thereby prevent the inner cylinder 50 from falling off from the rear end opening 45 of the outer cylinder 40. When the inner cylinder 50 pushes the tampon main body 20 out of the outer cylinder 40, the inner cylinder 50 moves in such a manner that the outer peripheral surface of the flange portion 51a comes into contact with the longitudinal ribs 54 of the major diameter portion 41. Further, as shown in Figs. 1 and 2, at the rear end side on an inner peripheral surface of the first inner cylinder 51, an annular protrusion 51b protruding inwardly in the radial direction of the first inner cylinder 51 is provided.

The second inner cylinder 52 is a cylindrical body made of a thermoplastic resin by injection molding. The second inner cylinder 52 has an external diameter that is slightly smaller than an internal diameter of the first inner cylinder 51. The second inner cylinder 52 is, when the inner cylinder 50 is in a contracted state, inserted in the first inner cylinder 51 as shown in Fig. 1 and, when the inner cylinder 50 is in an extended state, connected to the rear end part of the first inner cylinder 51 at the leading end part of the second inner cylinder 52 as shown in Fig. 2. On the outer peripheral surface of the leading end part of the second inner cylinder 52, an arcuate flange portion 52a and a protruded part 52b provided at a position nearer to the rear end side than the flange portion 52a are formed. As shown in Fig. 2, a height of the protruded part 52b gradually lowers towards the rear end. Note that a space between the flange portion 52a and the protruded part 52b of the second inner cylinder 52 is slightly greater than a thickness of the annular protrusion 51b of the first inner cylinder 51.

Then, as the second inner cylinder 52 is pulled towards the rear end side, the annular protrusion 51b of the first inner cylinder 51 will be at a position between the flange portion 52a and the protruded part 52b of the second inner cylinder 52. In such a state, as shown in Fig. 2, the annular protrusion 51b engages the flange portion 51a and the protruded part 52b, and thus the first inner cylinder 51 and the second inner cylinder 52 are connected.

Further, as shown in Figs. 1 and 2, a flared portion 52c is formed at the rear end part of the second inner cylinder 52. An external diameter of the flared portion 52c is preferably at least greater than the internal diameter of the first inner cylinder 51 and greater than or equal to the internal diameter of the minor diameter portion 42 of the outer cylinder 40.

As described above, in the tampon 10 according to this embodiment, the outer cylinder 40 has the longitudinal ribs 54 on its inner surface 40a along the longitudinal direction of the outer cylinder 40. Then, as a result, the tampon 10 in which the tampon main body 20 is pushed outside in a satisfactory manner is obtained.

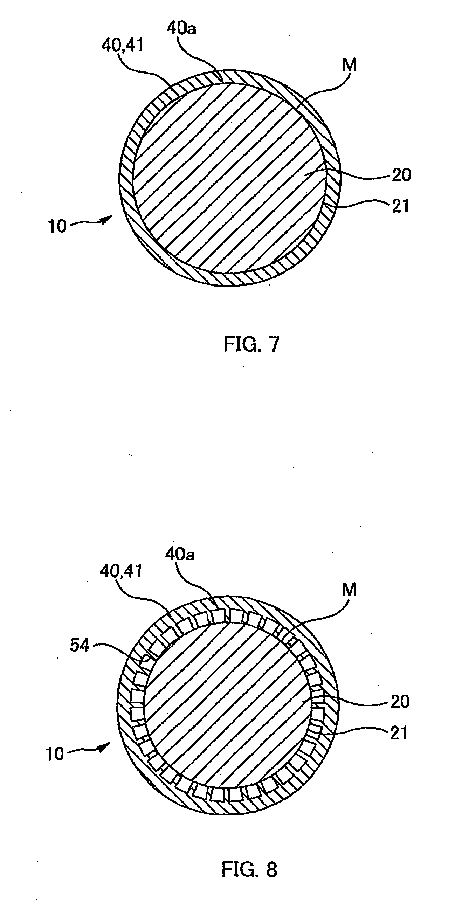

The above will be described using Fig. 7 and by comparing the tampon 10 according to this embodiment and a tampon 10 according to a comparative example. Fig. 7 is a diagram corresponding to Fig. 5 and shows the tampon 10 according to the comparative example.

When the tampon 10 according to this embodiment and the tampon 10 according to the comparative example are compared, they have a common feature that they include the tampon main body 20, the outer cylinder 40 that is for accommodating the tampon main body 20 and that has the leading end opening 43 at the leading end, and the inner cylinder 50 that is for pushing out the tampon main body 20 outside of the outer cylinder 40 from the leading end opening 43 by moving inside the outer cylinder 40. However, the tampon 10 according to the comparative example does not have the longitudinal ribs, and as is clear from Fig. 7, a contacting area of the outer surface 21 of the tampon main body 20 and the inner surface 40a of the outer cylinder 40 becomes large. Therefore, there is a case where at the time the inner cylinder 50 pushes the tampon main body 20 outside of the outer cylinder 40 from the leading end opening 43 by a user handling the inner cylinder 50, the tampon main body 20 is not pushed outside in a satisfactory manner due to an excessively large friction that occurs between the outer surface 21 and the inner surface 40a.

On the contrary, with the tampon 10 according to this embodiment, since the outer cylinder 40 has the longitudinal ribs 54 on its inner surface 40a along the longitudinal direction of the outer cylinder 40, the outer surface 21 of the tampon main body 20 and the longitudinal ribs 54 come into contact, and their contacting surfaces become significantly smaller compared with the comparative example, as is clear from Fig. 5. Therefore, at the time the outer cylinder 50 pushes out the tampon main body 20 outside of the outer cylinder 40 from the leading end opening 43 by the user handling the inner cylinder 50, the friction becomes smaller, and the tampon main body 20 is to be pushed outside in a satisfactory manner.

Further, the tampon 10 according to this embodiment has a merit that generation of static electricity is suppressed due to the friction becoming smaller.

Further, in this embodiment, the outer cylinder 40 has equal to or more than three longitudinal ribs 54 that are arranged at equal spacing in an inner peripheral direction of the inner surface 40a, so that the tampon main body 20 is to be completely supported by the longitudinal ribs 54. This makes it easy to create a situation where the tampon main body 20 contacts only the longitudinal ribs 54 (a situation where the tampon main body 20 does not contact the inner surface 40a). Therefore, at the time the tampon main body 20 is pushed outside of the outer cylinder 40, it is certainly possible to make the friction smaller, and the tampon main body 20 is to be pushed outside in a more satisfactory manner.

Further, in this embodiment, since the tampon main body 20 is to contact only the longitudinal ribs 54, of the inner surface 40a and the longitudinal ribs 54, at the time the tampon main body 20 is pushed outside of the outer cylinder 40, the friction certainly becomes smaller, and the tampon main body 20 is pushed outside in a more satisfactory manner.

Further, in this embodiment, since the outer cylinder 40 has the longitudinal ribs 54 at least at the leading end side from the center C in the longitudinal direction of the outer cylinder 40, there are the below improvements. That is, while the tampon main body 20 has started to be pushed outside of the outer cylinder 40 until it has finished being pushed outside, more of the tampon main body 20 slides in the portion at the leading end side from the center C than in the portion at the rear end side from the center C (That is, only the rear end side of the tampon main body 20 slides in the portion at the rear end side than the center C, but the rear end side as well as the leading end side of the tampon main body 20 slides in the portion at the leading end side from the center C.). Therefore, if the outer cylinder 40 has the longitudinal ribs 54 at least at the leading end side from the center C of the outer cylinder 40, the tampon main body 20 is to be pushed outside in a more satisfactory manner.

In this embodiment, since the outer cylinder 40 does not have the longitudinal ribs 54 in the petaloid portion 44, there are the following improvements. That is, supposing that in the case that the longitudinal ribs 54 are provided in the petaloid portion 44, the rigidity of the petaloid portion 44 increases, and it becomes harder for the leading end opening 43 to open. Therefore, there is a possibility that the tampon main body 20 will not be pushed outside in a satisfactory manner. In this embodiment, since there are no longitudinal ribs 54 in the petaloid portion 44, a problem that it becomes hard for the leading end opening 43 to open does not occur, and the tampon main body 20 is pushed outside in a more satisfactory manner.

Further, in this embodiment, the tampon main body 20 is accommodated in only the major diameter portion 41, of the major diameter portion 41 and the minor diameter portion 42, and the inner cylinder 50 pushes out the tampon main body 20 outside of the outer cylinder 40 from the leading end opening 43 by moving inside the major diameter portion 41 and the minor diameter portion 42 of the outer cylinder 40, and the outer cylinder 40 has the longitudinal ribs 54 in only the major diameter portion 41 of the major diameter portion 41 and the minor diameter portion 42. Thus, the longitudinal ribs 54 are provided in only the portions that are necessary in which the tampon main body 20 is accommodated, and therefore there is an improvement in that the tampon main body will be efficiently pushed out in a satisfactory manner.

Further, in this embodiment, the projection height h of the longitudinal ribs 54 in the radial direction of the major diameter portion 41 is made smaller than a difference in internal diameters R-r of the major diameter portion 41 and the minor diameter portion 42. That is, the rib-considering internal diameter R-h of the major diameter portion 41 is made larger than an internal diameter r of the minor diameter portion 42. Thus, a problem that the tampon main body 20 accommodated in the major diameter portion 41 enters the minor diameter portion 42 occurs is prevented.

Further, in this embodiment, the product M is applied on the outer surface 21 of the tampon main body 20, so that the structure in which the outer cylinder 40 has on its inner surface 40a the longitudinal ribs 54 along the longitudinal direction of the outer cylinder 40 creates the following effects. That is, supposing that in the case the longitudinal ribs are not provided, the contacting surface of the outer surface 21 of the tampon main body 20 and the inner surface 40a of the outer cylinder 40 becomes large. Thus, at the time the tampon main body 20 is pushed outside of the outer cylinder 40, a large amount of the product M applied on the outer surface 21 moves to the inner surface 40a of the outer cylinder 40. Therefore, the amount of the product M that is administered into the vaginal cavity becomes less. On the contrary, this embodiment has the above structure so that the contact surface between the outer surface 21 and the inner surface 40a becomes smaller. Thus, at the time the tampon main body 20 is pushed outside of the outer cylinder 40, the amount of the product that moves from the outer surface 21 to the inner surface 40a lessens, so that the problem that the amount of product M that is administered into the vaginal cavity lessens can be avoided.

### === Other embodiments ===

The tampon according to the present invention has been described with reference to the above embodiment. However, the above-described embodiment is provided for the purpose of facilitating the understanding of the present invention only and does not give any limitation to the present invention.

Further, in the above embodiment, the width of the longitudinal ribs 54 is narrowed the closer it gets to the center in the radial direction, however, it is not limited to such. For example, as shown in Fig. 8, the width of the longitudinal ribs 54 may be the same across the radial direction. However, the longitudinal ribs 54 according to the above embodiment are more preferable in that the above contacting surface can be made smaller. Fig. 8 is a diagram corresponding to Fig. 5, and shows longitudinal ribs 54 corresponding to another embodiment.

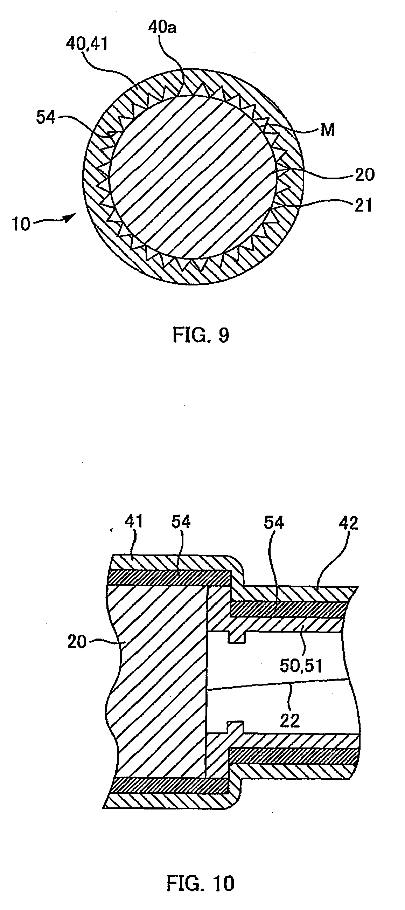

Further, in this embodiment, the longitudinal ribs 54 next to each other do not contact each other (are not bordering each other), however it is not limited to such, and as shown in Fig. 9, the longitudinal ribs 54 next to each other may be contacting each other. Fig. 9 is a diagram corresponding to Fig. 5, and shows the longitudinal ribs 54 according to another embodiment.

Further, in this embodiment, the outer cylinder 40 has the longitudinal ribs 54 in only the major diameter portion 41, of the major diameter portion 41 and the minor diameter portion 42. Fig. 10 shows a tampon that does not form an embodiment of the claimed invention. In Fig. 10 both the maj or diameter portion 41 and the minor diameter portion 42 may have the longitudinal ribs 54. In this case, as is clear form Fig. 10, the inner cylinder 50 (the first inner cylinder 51) and the longitudinal ribs 54 of the minor diameter portion 42 come into contact, and both contact surfaces become significantly smaller. Thus, at the time the inner cylinder 50 pushes out the tampon main body 20 outside of the outer cylinder 40 from the leading end opening 43 by the user handling the inner cylinder 50, the friction between the inner cylinder 50 and the longitudinal ribs 54 becomes small, and the inner cylinder 50 is to be appropriately moved. Fig. 10 is a diagram corresponding to Fig. 6.

The pine bark extract is given as an example of a constituent of the product M, but it is not limited to such. For example, it may be plant extracts, for example, such as red clover, anil extract, and indirubin. Further, Flavangenol is given as an example of a pine bark extract, but it is not limited to such. For example, Pycnogenole distributed by Nihon SiberHegner or Enzogenol distributed by Valentine Company Limited may be used.

### Reference Signs List

- 10: tampon,
- 20: tampon main body, 21 outer surface, 22 extraction cord, 30 applicator,
- 40: outer cylinder, 40a inner surface, 41 major diameter portion,
- 42: minor diameter portion, 43 leading end opening (opening),
- 44: petaloid portions, 45 rear end opening,
- 46: annular rib, 47 annular stepped portion,
- 50: inner cylinder, 51 first inner cylinder,
- 51a: annular flange portion, 51b annular protrusion,
- 52: second inner cylinder, 52a arcuate flange portion,
- 52b: protruded part, 52c flared portion,
- 54: longitudinal ribs, C Center, E1 foremost end, E2 rearmost end, M product

## Claims

1. A tampon (10) comprising:
a tampon main body (20);
an accommodating cylinder (40) that accommodates the tampon main body, the accommodating cylinder having an opening (43) at a leading end; and
a pushing member (50) that pushes out the tampon main body outside of the accommodating cylinder from the opening, by moving inside the accommodating cylinder,
wherein the accommodating cylinder has ribs (54) along a longitudinal direction of the accommodating cylinder on an inner surface (40a) of the accommodating cylinder,
wherein the accommodating cylinder comprises
a major diameter portion (41) positioned at the leading end side, the major diameter portion being formed with the opening, and
a minor diameter portion (42) positioned at a rear end side opposite to the leading end side, the minor diameter portion having an internal diameter smaller than that of the major diameter portion,
the tampon main body is accommodated in only the major diameter portion, of the major diameter portion and the minor diameter portion,
the pushing member pushes out the tampon main body outside of the accommodating cylinder from the opening, by moving inside the major diameter portion and the minor diameter portion of the accommodating cylinder, and
the accommodating cylinder has the ribs in only the major diameter portion, of the major diameter portion and the minor diameter portion,
the accommodating cylinder has a plurality of petaloid portions (44) surrounding the opening,
the ribs are formed from a rearmost end (E2) of the major diameter portion to a foremost end (E1) of the major diameter portion excluding the petaloid portions, and
a projection height (h) of the ribs in a radial direction of the major diameter portion is smaller than an internal diameter difference between the major diameter portion and the minor diameter portion.

2. A tampon according to claim 1,
wherein the accommodating cylinder has greater than or equal to three ribs arranged at equal spacing in an inner peripheral direction of the inner surface.

3. A tampon according to claim 2,
wherein the tampon main body is in contact with only the ribs, of the inner surface and the ribs.

4. A tampon according to any of claims 1 to 3,
wherein the accommodating cylinder has the ribs at least at the leading end side from the center in the longitudinal direction of the accommodating cylinder.

5. A tampon according to any of claims 1 to 4,
wherein a product is applied on an outer surface (21) of the tampon main body.

## Patentansprüche

1. Tampon (10), der Folgendes umfasst:
einen Hauptkörper (20) des Tampons,
einen anpassenden Zylinder (40), der den Hauptkörper des Tampons unterbringt, wobei der anpassende Zylinder eine Öffnung (43) am vorderen Ende hat, und
ein schiebendes Element (50), das den Hauptkörper des Tampons außerhalb des anpassenden Zylinders von der Öffnung hinaus schiebt, indem es in den anpassenden Zylinder geschoben wird"
wobei der anpassende Zylinder Rippen (54) in Längsrichtung des anpassenden Zylinders auf einer Innenfläche (40a) des anpassenden Zylinders hat,
wobei der anpassende Zylinder Folgendes umfasst:
einen hauptsächlichen Durchmesserabschnitt (41) der auf der Seite des vorderen Ende positioniert ist, wobei der hauptsächliche Durchmesserabschnitt mit der Öffnung geformt wird, und
einen nebensächlichen Durchmesserabschnitt (42), der an einer Seite des hinteren Endes gegenüber der Seite des vorderen Endes positioniert ist, wobei der nebensächliche Durchmesserabschnitt einen internen Durchmesser hat, der kleiner ist, als der des hauptsächlichen Durchmesserabschnitts,
der Hauptkörper des Tampons nur im hauptsächlichen Durchmesserabschnitt des hauptsächlichen Durchmesserabschnitts und des nebensächlichen Durchmesserabschnitts untergebracht wird,
wobei das schiebende Element den Hauptkörper des Tampons aus dem anpassenden Zylinder von der Öffnung schiebt, indem es in den hauptsächlichen Durchmesserabschnitt und den nebensächlichen Durchmesserabschnitt des anpassenden Zylinders geht, und
der anpassende Zylinder die Rippen nur im hauptsächlichen Durchmesserabschnitt, vom hauptsächlichen Durchmesserabschnitt und dem nebensächlichen Durchmesserabschnitt hat,
der anpassende Zylinder eine Vielzahl von petaloiden Abschnitten (44) hat, die die Öffnung umgeben,
die Rippen von einem hintersten Ende (E2) des hauptsächlichen Durchmesserabschnitts zu einem vordersten Ende (E1) des hauptsächlichen Durchmesserabschnitts, mit Ausnahme der petaloiden Abschnitte geformt sind, und
eine Projektionshöhe (h) der Rippen in radialer Richtung des hauptsächlichen Durchmesserabschnitts kleiner ist, als ein interner Durchmesserunterschied zwischen dem hauptsächlichen Durchmesserabschnitt und dem nebensächlichen Durchmesserabschnitt.

2. Tampon nach Anspruch 1,
wobei der anpassende Zylinder größer als oder gleich drei Rippen ist, die mit gleichmäßigen Abständen in innerer peripherer Richtung der Innenfläche angeordnet sind.

3. Tampon nach Anspruch 2,
wobei der Hauptkörper des Tampons nur mit den Rippen in Berührung ist, von der Innenfläche und den Rippen.

4. Tampon nach einem der Ansprüche 1 bis 3,
wobei der anpassende Zylinder die Rippen wenigstens auf der Seite des vorderen Ende von der Mitte in Längsrichtung des anpassenden Zylinders hat.

5. Tampon nach einem der Ansprüche 1 bis 4,
wobei ein Produkt auf einer Außenseite (21) des Hauptkörpers des Tampons eingesetzt wird.

## Revendications

1. Tampon hygiénique (10) comportant :
un corps principal de tampon (20) ;
un cylindre de réception (40) qui reçoit le corps principal de tampon, le cylindre de réception ayant une ouverture (43) au niveau d'une extrémité avant ; et
un élément de poussée (50) qui pousse sur le corps principal de tampon pour le faire sortir à l'extérieur du cylindre de réception depuis l'ouverture, par déplacement à l'intérieur du cylindre de réception,
dans lequel le cylindre de réception a des nervures (54) le long d'une direction allant dans le sens longitudinal du cylindre de réception sur une surface intérieure (40a) du cylindre de réception,
dans lequel le cylindre de réception comporte
une partie à plus grand diamètre (41) positionnée au niveau du côté d'extrémité avant, la partie à plus grand diamètre étant formée avec l'ouverture, et
une partie à plus petit diamètre (42) positionnée au niveau d'un côté d'extrémité arrière à l'opposé du côté d'extrémité avant, la partie à plus petit diamètre ayant un diamètre intérieur inférieur à celui de la partie à plus grand diamètre,
le corps principal de tampon est reçu dans uniquement la partie à plus grand diamètre, parmi la partie à plus grand diamètre et la partie à plus petit diamètre,
l'élément de poussée pousse sur le corps principal de tampon pour le faire sortir à l'extérieur du cylindre de réception depuis l'ouverture, par déplacement à l'intérieur de la partie à plus grand diamètre et de la partie à plus petit diamètre du cylindre de réception, et
le cylindre de réception a les nervures dans uniquement la partie à plus grand diamètre, parmi la partie à plus grand diamètre et la partie à plus petit diamètre,
le cylindre de réception a une pluralité de parties pétaloïdes (44) entourant l'ouverture,
les nervures sont formées depuis une extrémité se trouvant le plus en arrière (E2) de la partie à plus grand diamètre jusqu'à une extrémité se trouvant le plus en avant (E1) de la partie à plus grand diamètre à l'exclusion des parties pétaloïdes, et
une hauteur en saillie (h) des nervures dans une direction allant dans le sens radial de la partie à plus grand diamètre est inférieure à une différence de diamètre interne entre la partie à plus grand diamètre et la partie à plus petit diamètre.

2. Tampon hygiénique selon la revendication 1,
dans lequel le cylindre de réception a un nombre supérieur ou égal à trois nervures agencées de manière équidistante dans une direction allant dans le sens de la périphérie intérieure de la surface intérieure.

3. Tampon hygiénique selon la revendication 2,
dans lequel le corps principal de tampon est en contact avec uniquement les nervures, parmi la surface intérieure et les nervures.

4. Tampon hygiénique selon l'une quelconque des revendications 1 à 3,
dans lequel le cylindre de réception a les nervures au moins au niveau du côté d'extrémité avant depuis le centre dans la direction allant dans le sens longitudinal du cylindre de réception.

5. Tampon hygiénique selon l'une quelconque des revendications 1 à 4,
dans lequel un produit est appliqué sur une surface extérieure (21) du corps principal de tampon.
